# EUROPEAN PATENT APPLICATION

(11) **EP 1 577 284 A1**
(43) Date of publication of application: **21.09.2005**
(21) Application number: 03775968.5
(22) Date of filing: 28.11.2003
(51) Int. Cl.: C07C 43/12, C07C 41/09

(54) **FLUORINE-CONTAINING ETHER COMPOUND AND METHOD FOR PRODUCING SAME**

(30) Priority: 24.12.2002 JP 2002372882
(71) Applicant: ASAHI GLASS COMPANY LTD., Tokyo 100-8405 (JP)
(72) Inventor: OHARU, Kazuya Asahi Glass Company, Limited, Kanagawa 221-8755 (JP); TAKAGI, Hirokazu Asahi Glass Company, Limited, Kanagawa 221-8755 (JP); SIMPSON, Roderic Nigel Fraser Honeysuckle Cottage, Mon NP16 7JW (GB)
(74) Representative: Müller-Boré & Partner Patentanwälte
(86) International application number: PCT/JP2003/015279
(87) International publication number: WO 2004/058674

(57) **Abstract**

This invention provides a novel fluorinated ether compound represented by the following formula (I), which has a short atmospheric lifetime and which is stable under use conditions. The fluorinated ether compound of the invention is useful as a fluorinated inert medium or the like.

R^{f}(CH₂)ₙO(CH₂)ₙR^{f} (I)

wherein R^{f} is a C₁₋₁₀ fluorinated monovalent saturated organic group, and n is 3 or 4.

## Description

### TECHNICAL FIELD

The present invention relates to a novel fluorinated ether compound useful as an inert medium, etc., and a method for producing such a compound.

### BACKGROUND ART

As fluorinated inert media, fluorocarbons are mainly used. Specifically, they are used as insulating oils in the field of electronics, as media being used for thermal shock tests or leak tests, as oxygen carriers being used for surgery in the biomedical field, or as cleaning agents or water repellents in the industrial fields. Such fluorocarbons can, for example, be perfluoroalkanes, perfluoroalkylamines or perfluorocyclic ethers, which are represented by the following formulae (see "Newest Aspect of Fluoro Functional Material" compiled by Masaaki Yamabe and Masashi Matsuo, published by CMC, 1994, p. 172-176).

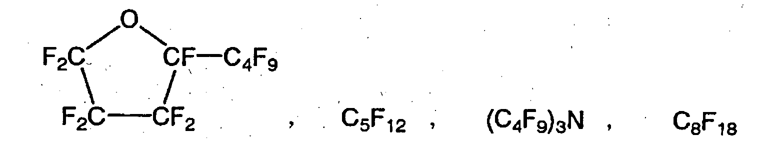

However, the above fluorocarbons have long atmospheric lifetimes and large global warming potentials (GWP). Accordingly, there is a possibility that their use will be substantially restricted in the future.

The purpose of the present invention is to provide a novel fluorinated ether compound which has a short atmospheric lifetime and which is yet stable under use conditions, and a method for producing such a compound.

### DISCLOSURE OF THE INVENTION

To solve the problems mentioned above, the present inventors have conducted an extensive research for a fluorinated ether compound which has a short atmospheric lifetime and which is stable under use conditions. As a result, it has been found that a fluorinated ether compound having a novel structure, represented by the following formula (I) is excellent in a cleaning property and stability since the fluoroalkyl moiety and the hydrocarbon moiety are completely separated, and the -CH₂OCH₂- structure is so reactive that the atmospheric lifetime is short.

Namely, the present invention relates to the following invention which solves the above problems.
(1) A fluorinated ether compound represented by the following formula (I):

   R^{f}(CH₂)ₙO(CH₂)ₙR^{f} (I)

   wherein R^{f} is a C₁₋₁₀ fluorinated monovalent saturated organic group, and n is 3 or 4.
(2) The fluorinated ether compound according to (1), wherein R^{f} is a C₁₋₁₀ polyfluoroalkyl group.
(3) The fluorinated ether compound according to (1), wherein R^{f} is a C₁₋₁₀ perfluoroalkyl group.
(4) The fluorinated ether compound according to (1), wherein R^{f} is -C₂F₅ or -C₈F₁₇.
(5) The fluorinated ether compound according to any one of (1) to (4), wherein n is 3.
(6) The fluorinated ether compound according to (1), wherein R^{f} is -C₂F₅, and n is 3.
(7) A method for producing a compound represented by the following formula (I), characterized in that an alcohol compound represented by the following formula (II) is subjected to a dehydration condensation reaction in the presence of a Lewis acid catalyst:

   R^{f}(CH₂)ₙOH (II)

   R^{f}(CH₂)ₙO(CH₂)ₙR^{f} (I)

   wherein R^{f} is a C₁₋₁₀ fluorinated monovalent saturated organic group, and n is 3 or 4.
(8) The method according to (7), wherein R^{f} is a C₁₋₁₀ polyfluoroalkyl group.
(9) The method according to (8), wherein R^{f} is -C₂F₅ or -C₈F₁₇, and n is 3.
(10) The method according to (7), (8) or (9), wherein the Lewis acid catalyst is a perfluoroalkane sulfonic acid.

### BEST MODE FOR CARRYING OUT THE INVENTION

In the present invention, the group represented by R^{f} (hereinafter referred to as the R^{f} group) is a C₁₋₁₀ fluorinated monovalent saturated organic group. The carbon number of the R^{f} group is preferably from 1 to 8, more preferably from 1 to 4. The R^{f} group may be linear or branched or may form a ring. The R^{f} group comprises carbon atoms and fluorine atoms essentially and may contain hydrogen atoms, oxygen atoms, nitrogen atoms, or halogen atoms other than fluorine atoms.

The monovalent saturated organic group in the C₁₋₁₀ fluorinated monovalent saturated organic group (the R^{f} group), may be an alkyl group, a cycloalkyl group, an alkyl group containing a hetero atom, or a cycloalkyl group containing a hetero atom. The hetero atom in the alkyl group containing a hetero atom or the cycloalkyl group containing a hetero atom, is preferably an oxygen atom or a nitrogen atom, particularly preferably an oxygen atom, especially preferably an etheric oxygen atom.

In the case where the alkyl group containing a hetero atom is an alkyl group containing an etheric oxygen atom, the number of the etheric oxygen atom may be one, or two or more. Further, the etheric oxygen atom is preferably present between carbon-carbon atoms in an alkyl group, or at the bond terminal of the alkyl group.

In the case where the cycloalkyl group containing a hetero atom is a cycloalkyl group having an etheric oxygen atom, the number of the etheric oxygen atom may be one, or two or more. Further, such an etheric oxygen atom is preferably present between carbon-carbon atoms in the cycloalkyl group.

The C₁₋₁₀ fluorinated monovalent saturated organic group may contain halogen atoms other than fluorine atoms. The halogen atoms other than fluorine atoms are preferably chlorine atoms.

In the case where the R^{f} group is a group containing a trifluoromethyl group (such as a group of e.g. CF₃(CF₂)ₘ- or (CF₃)₂CF(CF₂)ₖ-, where m is an integer of from 0 to 9, and k is an integer of from 0 to 7), the surface tension tends to be low, such being preferred. Further, the terminal carbon atom bonded to -(CH₂)ₙ of the R^{f} group is preferably a carbon atom to which at least one fluorine atom is bonded.

The mass ratio of fluorine atoms (the fluorine content) in the R^{f} group is preferably at least 40 mass%. Further, the R^{f} group is particularly preferably a perfluoroalkyl group i.e. a group constituted solely by carbon atoms and fluorine atoms, and especially preferred is a linear perfluoroalkyl group.

The following groups may be mentioned as specific examples of the R^{f} group.
- C₂F_{5,} -C₃F₇ (such as -(CF₂)₃F or -CF(CF₃)₂), -C₈F₁₇ (such as -(CF₂)₈F), -C₂HF₄ (such as -CHFCF₃), -C₂ClF₄ (such as -CClFCF₃), -CF(CF₃)OC₃F₇ (such as -CF(CF₃)O(CF₂)₃F).

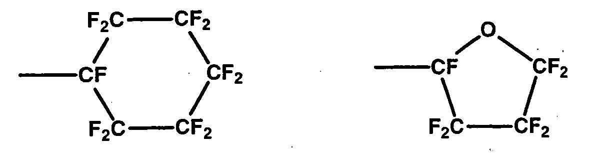

The R^{f} group is preferably a group selected from a C₁₋₁₀ polyfluoroalkyl group, a C₃₋₁₀ polyfluorocycloalkyl group, a C₁₋₁₀ polyfluoroalkyl group having an etheric oxygen atom and a C₃₋₁₀ polyfluorocycloalkyl group having an etheric oxygen atom. When hydrogen atoms are present in such a selected group, it is preferred that some or all of such hydrogen atoms are substituted by chlorine atoms. Particularly preferred is a C₁₋₁₀ perfluoroalkyl group, a C₃₋₁₀ perfluorocycloalkyl group, a C₁₋₁₀ perfluoroalkyl group having an etheric oxygen atom, or a C₃₋₁₀ perfluorocycloalkyl group having an etheric oxygen atom. Especially preferred is a C₁₋₁₀ perfluoroalkyl group. Further, as the R^{f} group, -C₂F₅ or -C₈F₁₇ is preferred.

Further, n in the formula (I) is 3 or 4, and n is preferably 3.

The following compounds may be mentioned as specific examples of the fluorinated ether compound of the present invention. However, when the following compounds have structural isomers wherein the structures of the perfluoroalkyl group moieties are different, the following examples include all such structural isomers.

C₂ F₅ CH₂ CH₂ CH₂ OCH₂ CH₂ CH₂ C₂ F₅,

C₃ F₇ CH₂ CH₂ CH₂ OCH₂ CH₂ CH₂ C₃ F₇,

(CF₃)₂ CFCH₂ CH₂ CH₂ OCH₂ CH₂ CH₂ CF (CF₃)₂,

C₈ F₁₇ CH₂ CH₂ CH₂ OCH₂ CH₂ CH₂ C₈F₁₇.

Each of the fluorinated ether compounds represented by the formula (I) of the present invention is a novel compound which has not been disclosed in any literature. The compound represented by the formula (I) can easily be prepared by using as a starting material a fluorinated alcohol compound represented by the following formula (II) which is industrially readily available and by subjecting such a fluorinated alcohol compound to a dehydration condensation reaction in the presence of a Lewis acid catalyst.

R^{f}(CH₂)ₙOH (II)

wherein R^{f} and n are as defined above.

The Lewis acid catalyst to be used for the dehydration condensation reaction, may, for example, be phosphoric acid, polyphosphoric acid, sulfuric acid, p-toluenesulfonic acid, a perfluoroalkane sulfonic acid or a perfluorocarboxylic acid. As the Lewis acid catalyst, from the viewpoint of the reactivity and selectivity, it is preferred to employ sulfuric acid or a sulfonic acid type compound, and it is particularly preferred to employ a perfluoroalkane sulfonic acid.

From the viewpoint of the reactivity and economical efficiency, the amount of the Lewis acid catalyst is preferably from 0.001 to 1.0 time by mol, particularly preferably from 0.01 to 0.2 time by mol, to the fluorinated alcohol compound as the starting material.

A reaction solvent is not essential in the dehydration condensation reaction, but it may be used as the case requires. In a case where a reaction solvent is to be used, it is preferred to use a solvent which is inert to the reaction, and for example, a perfluorocarbon, a perfluorohydrocarbon or a fluorochlorohydrocarbon is particularly preferred.

The dehydration condensation reaction is preferably carried out while removing water formed. However, even without removing water, it is possible to obtain a reaction conversion at a level of 70%.

The temperature for the dehydration condensation reaction may suitably be changed depending upon the type of the starting material compound or the inert solvent to be used, or other conditions, and it is usually preferably from 100 to 250°C, particularly preferably from 150 to 200°C.

The reaction mixture formed by the dehydration condensation reaction is usually subjected to purification treatment depending upon the particular purpose to isolate the desired fluorinated ether compound. The isolation method is preferably a distillation method.

The compound of the present invention is a compound having a short atmospheric lifetime. The atmospheric lifetime of the compound of the present invention results from the reactivity with OH radicals, whereby the atmospheric lifetime is found to be not more than 0.1 year.

### EXAMPLES

Now, the present invention will be described in detail with reference to Examples. However, it should be understood that the present invention is by no means restricted by such Examples.

### EXAMPLE 1

Into a 1L hastelloy C autoclave, pentafluoropentanol (C₂F₅(CH₂)₃OH, 519 g) and trifluoromethane sulfonic acid (20 g) were charged and stirred at 175°C for 14 hours. The reactor pressure at that time was 0.7 MPa (gage pressure). After the reaction, the obtained crude liquid was subjected to liquid separation, and the organic phase was washed twice with water (50 ml) and dried over magnesium sulfate, followed by filtration to obtain a crude liquid. The conversion by the reaction was 75.7% as measured by gas chromatography (hereinafter referred to simply as GC). By distillation under reduced pressure, the desired following compound (I-1) (172 g) was obtained as a fraction of (60 to 61°C)/1.4 kPa (absolute pressure). The GC purity was 99.9%. By the NMR analysis, formation of the following compound (I-1) was confirmed.

Compound (I-1) : C₂F₅(CH₂)₃O(CH₂)₃C₂F₅.
¹HNMR (300.4MHz, CDCl₃, TMS) δ (ppm) : 1.87 (m, 2H), 2.12 (m, 2H), 3.48 (m, 2H).
¹⁹FNMR (282.7MHz, CDCl₃, CFCl₃) δ (ppm) : -86.1 (3F), -118.8(2F).

### EXAMPLE 2

Heptadecafluorooctylpropanol (F(CF₂)₈(CH₂)₃OH, 252 g) and trifluoromethane sulfonic acid (2 g) were put into a 200 ml four-necked flask and vigorously stirred at 180°C for 5 hours. At that time, the reaction was carried out while withdrawing water formed in the reaction. After completion of the reaction, 200 g of dichloropentafluoropropane ("AK225" tradename, manufactured by Asahi Glass Company, Limited) was added. The obtained crude liquid was subjected to liquid separation, and the organic phase was washed twice with water (50 mL) and dried over magnesium sulfate, followed by filtration, to obtain a crude liquid. The conversion by the reaction was 97.2% as measured by GC. By removing low boiling point compounds, the desired following compound (I-2) (206 g) was obtained. The GC purity was 98.1%. By the NMR analysis, formation of the following compound (I-2) was confirmed.

Compound (I-2) : F(CF₂)₈(CH₂)₃O(CH₂)₃(CF₂)₈F.
¹HNMR(300.4MHz, CDCl₃ TMS) δ (ppm):1.97 (m, 2H), 2.18 (m, 2H), 3.58 (t, 2H).
¹⁹FNMR(282. 7MHz, CDCl₃, CFCl₃) δ (ppm) : -81.3(3F), -114.9(2F), -122.2(2F), -122.4(4F), -123.2(2F), -123.9(2F), -126.6(2F).

Further, with respect to the compound (I-1) obtained in Example 1 and the compound (I-2) obtained in Example 2, the following tests were carried out.

### EXAMPLE 3: Example of stability test

With reference to JIS K-1508, 200 ml each of the test samples containing the compound (I-1) obtained in Example 1 and the compound (I-2) obtained in Example 2, respectively, was prepared. A piece of soft steel was put therein, and while supplying oxygen, light of 30000 lux was irradiated for 48 hours. Each of the compounds (I-1) and (I-2) of the present invention did not decompose, and the stability was good. As a control substance, perfluoro(2-butyltetrahydrofuran) was used, and the same test was carried out, whereby the stability was good.

### EXAMPLE 4: Example of cleaning test

A stainless steel test piece (SUS-304, 25 mm × 30 mm × 2 mm) having temper oil (manufactured by NIPPON GREASE Co., Ltd.) deposited thereon was subjected to warm bath ultrasonic cleaning by using each of the compound (I-1) obtained in Example 1 and the compound (I-2) obtained in Example 2. The appearance of the test piece after cleaning was visually inspected, whereby no remaining oil was observed, and thus the compounds of the present invention exhibited good cleaning properties. On the other hand, the cleaning property of perfluoro(2-butyltetrahydrofuran) used as the control substance was poor, and the remaining oil was observed.

### INDUSTRIAL APPLICABILITY

The novel fluorinated ether compound provided by the present invention is useful as a fluorinated inert medium. The fluorinated ether compound is useful as an insulating oil in the electronics field, as a medium to be used for a thermal shock test, a leak test, etc., an oxygen carrier to be used for surgery in the biomedical field, or as a cleaning agent or water repellent in the industrial field. Further, it is useful also as a compatibilizing agent for a hydrocarbon compound and a fluorocarbon compound.

## Claims

1. A fluorinated ether compound represented by the following formula (I):
R^{f}(CH₂)ₙO(CH₂)ₙR^{f} (I)
wherein R^{f} is a C₁₋₁₀ fluorinated monovalent saturated organic group, and n is 3 or 4.

2. The fluorinated ether compound according to Claim 1, wherein R^{f} is a C₁₋₁₀ polyfluoroalkyl group.

3. The fluorinated ether compound according to Claim 1, wherein R^{f} is a C₁₋₁₀ perfluoroalkyl group.

4. The fluorinated ether compound according to Claim 1, wherein R^{f} is -C₂F₅ or -C₈F₁₇.

5. The fluorinated ether compound according to any one of Claims 1 to 4, wherein n is 3.

6. The fluorinated ether compound according to Claim 1, wherein R^{f} is -C₂F₅, and n is 3.

7. A method for producing a compound represented by the following formula (I), **characterized in that** an alcohol compound represented by the following formula (II) is subjected to a dehydration condensation reaction in the presence of a Lewis acid catalyst:
R^{f}(CH₂)ₙOH (II)
R^{f}(CH₂)ₙO(CH₂)ₙR^{f} (I)
wherein R^{f} is a C₁₋₁₀ fluorinated monovalent saturated organic group, and n is 3 or 4.

8. The method according to Claim 7, wherein R^{f} is a C₁₋₁₀ polyfluoroalkyl group.

9. The method according to Claim 8, wherein R^{f} is -C₂F₅ or -C₈F₁₇, and n is 3.

10. The method according to Claim 7, 8 or 9, wherein the Lewis acid catalyst is a perfluoroalkane sulfonic acid.
